# EUROPÄISCHE PATENTSCHRIFT

(11) **EP 2 910 221 B1**
(45) Veröffentlichungstag und Bekanntmachung des Hinweises auf die Patenterteilung: **29.11.2017**
(21) Anmeldenummer: 15151808.1
(22) Anmeldetag: 20.01.2015
(51) Int. Cl.: A61F 2/966, A61F 2/97, A61F 2/95

(54) **Freigabevorrichtung zum Lösen eines medizinischen Implantats von einem Katheter sowie Katheter mit einer Freigabevorrichtung**
Release device for releasing a medical implant from a catheter, and catheter comprising a release device
Dispositif de dégagement pour libérer un implant médical d'un cathéter ainsi que cathéter avec dispositif de dégagement

(30) Priorität: 25.02.2014 US 201461944078 P
(43) Veröffentlichungstag der Anmeldung: 26.08.2015
(73) Patentinhaber: Biotronik AG, 8180 Bülach (CH)
(72) Erfinder: Fargahi, Amir, 8180 Bülach (CH)
(74) Vertreter: Randoll, Sören

(56) Entgegenhaltungen:
- EP-A1- 1 844 739
- EP-A1- 2 111 826
- WO-A1-2007/022395
- WO-A1-2010/120670
- WO-A1-2010/120671
- US-A1- 2005 080 476

## Beschreibung

Die Erfindung betrifft eine Freigabevorrichtung zum Lösen eines medizinischen Implantats von einem Katheter sowie einen Katheter mit einer Freigabevorrichtung

In der Medizin kommen häufig Implantate zum Einsatz, die zur Erfüllung von Ersatzfunktionen permanent oder zumindest für einen längeren Zeitraum in einen tierischen und/oder menschlichen Körper eingebracht werden. Zu nennen wären hier beispielsweise Herzschrittmacher, Hirnschrittmacher für Parkinsonpatienten, Herzimplantate, Cochleaimplantate, Retina-Implantate, zahnmedizinische Implantate, Implantate zum Gelenkersatz, Gefäßprothesen oder Stents.

Implantate sind zum Einführen in den Körper mit Kathetern verbunden und müssen am Einsatzort genau platziert und definiert freigegeben werden können. Hierzu ist beispielsweise bekannt, das Implantat durch eine Schiebbewegung freizugeben.

Dabei besteht bei bekannten Kathetern eine Abhängigkeit zwischen der Gesamtlänge des Katheters und der Länge des Implantats, beispielsweise eines Stents. Je länger der Stent ist, desto länger wird die Gesamtlänge des Katheters. So ist die Gesamtlänge für einen Katheter zum Einführen eines Stents von 200 mm Länge um mindestens 180 mm länger als derselbe Katheter mit einem Stent von 20 mm Länge. Dies erschwert die Freigabe des Implantats am vorgesehenen Ort.

Für den Arzt bedeutet dies den Einsatz eines längeren Führungsdrahts, was beim Freigeben des Implantats zu einem längeren Verschiebungsweg des Katheters über den Führungsdraht und zu einer komplizierten Handhabung führt. Ergonomisch gesehen wird die Handhabung eines derartigen langen Systems für den Anwender unangenehm. Der Anwender braucht beide Hände zur Freigabe des Implantats mit dem dazu vorgesehenen so genannten "Pull-Back-System" als Freigabevorrichtung.

Aus der EP 1 844 739 A1 ist ein Freigabesystem für einen langen Stent bekannt, das einen kurzen Handgriff aufweist. Um den Stent im Einsatzort freizugeben, wird ein Außenschaft zurückgezogen, wobei dieser durch einen Abzweig aus dem Handgriff weggeführt wird. In EP 2 111 826 A1 wird eine Einführvorrichtung offenbart. In WO 2007/022395 A1 wird ein System zum Einführen einer medizinischen Vorrichtung offenbart.
In WO 2010/120670A1 wird ein Einführsystem mit einem Schaft beschrieben, der einen Stent umgibt.

Der Erfindung liegt die Aufgabe zugrunde, eine Freigabevorrichtung anzugeben, die eine bequeme Handhabung auch bei langen Stents ermöglicht.

Eine weitere Aufgabe besteht in der Schaffung eines Katheters zum Einführen eines langen Stents mit einer Freigabevorrichtung.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen, der Beschreibung und der Zeichnung.

Es wird eine Freigabevorrichtung zum Lösen eines medizinisches Implantats von einer Einführvorrichtung vorgeschlagen, bei welcher das Implantat durch eine Relativbewegung zwischen einem ersten und einem zweiten Einführelement freisetzbar ist, wobei die Einführvorrichtung ein proximales Ende aufweist, das im Benutzungszustand einem Anwender zugewandt ist und ein distales Ende, das im Benutzungszustand vom Anwender entfernt ist. Zwischen dem proximalen und dem distalen Ende ist ein Verkürzungsbereich vorgesehen, in dem bei Erzeugung einer gezielten Relativbewegung zwischen dem ersten und dem zweiten Einführelement der Einführvorrichtung eine Länge wenigstens eines der Einführelemente zwischen dem proximalen und dem distalen Ende verkürzbar ist.

Das Verkürzen des Einführelements erfolgt mittels Zusammenfalten. Vorteilhaft erlaubt das Design der Freigabevorrichtung ein einfaches relatives Verschieben eines Einführelements, z.B. eines Außenschafts über ein Innenelement, z.B. einen Innenschaft. Das Einführelement, z.B. Außenschaft, kann mit axial gerichteten Schlitzen vorgeschlitzt sein und im proximalen Bereich zusammengefaltet und damit gekürzt werden. Vorteilhaft ist es nicht notwendig, den Außenschaft aus dem Handgriff herauszuziehen, was eine geeignete Abdichtung erfordern würde. Ferner ist eine Länge einer Einführvorrichtung mit einer solchen Freigabevorrichtung unabhängig von einer Länge des Implantats.

Die erfindungsgemäße Freigabevorrichtung findet z.B. Anwendung bei der Freisetzung von selbstexpandierenden Stentsystemen, zum Zurückziehen eines Schutzes von ballonexpandierenden Stents, oder zum Zurückziehen eines Schutzes von beschichteten Ballons.

Gemäß einer günstigen Ausgestaltung kann im Verkürzungsbereich eine Aufwickeleinrichtung vorgesehen sein. Diese lässt sich kompakt und Platz sparend an einem geeigneten proximalen Ort, z.B. in einem Handgriff, unterbringen. Das Einführelement, welches zur Freisetzung des Implantats verschoben, insbesondere vom distalen Ende in Richtung zum proximalen Ende hin zurückgezogen wird, verbleibt innerhalb der Freisetzungsvorrichtung.

Gemäß einer günstigen Ausgestaltung kann eine Zugeinrichtung vorgesehen sein, die an wenigstens einem der Einführelemente angreift, um die Relativbewegung zwischen dem ersten und dem zweiten Einführelement zu bewirken. Insbesondere kann die Zugeinrichtung einen Zugfaden aufweisen, der mit der Aufwickeleinrichtung aufwickelbar ist, wobei wenigstens das eine der Einführelemente zur Verkürzung seiner Länge in eine oder mehrere Falten legbar ist. Vorteilhaft kann ein vorgeschlitzter Außenschaft im proximalen Bereich zusammengefaltet und damit gekürzt werden, indem der Zugfaden, der am Außenschaft befestigt ist, zum proximalen Ende hin gezogen und aufgewickelt wird und den Außenschaft dabei mitnimmt, der sich dabei im vorgeschlitzten Bereich in Falten legen kann.

Gemäß einer günstigen Ausgestaltung kann die Aufwickeleinrichtung eine Umlenkeinrichtung aufweisen, die mit der Zugeinrichtung zusammenwirken kann. Insbesondere kann die Umlenkeinrichtung wenigstens zwei Umlenkrollen aufweisen. Günstigerweise kann eine Flaschenzuganordnung in der Freigabevorrichtung integriert sein. Je nach Zahl der Umlenkrollen kann dabei die aufzuwendende Kraft, mit der die Zugeinrichtung zu betätigen ist, in einer Weise reduziert werden, wie sie bei Flaschenzügen bekannt ist.

Gemäß einer günstigen Ausgestaltung kann wenigstens eines der Einführelemente zur Verkürzung seiner Länge aufwickelbar sein. Dies kann zusätzlich zur Faltung des vorgeschlitzten Einführelements vorgesehen sein. Dabei können zwei Aufwickeleinrichtungen vorgesehen sein, die jeweils eine Hälfte des vorgeschlitzten Einführelements aufnehmen

Gemäß einer günstigen Ausgestaltung kann die Aufwickeleinrichtung mit einem Handrad gekoppelt sein. Dieses lässt sich bequem mit einer Hand bedienen.

Gemäß einer günstigen Ausgestaltung kann die Aufwickeleinrichtung mit einem Motor gekoppelt sein. Vorteilhaft ist der Motor in einem Handgriff der Einführvorrichtung integriert und kann mit einem Start-Stop-Knopf am Handgriff bedient werden. Alternativ kann ein Aufwickelmechanismus vorgesehen sein, der mit Federkraft betätigt werden kann.

Nach einem weiteren Aspekt der Erfindung wird eine Einführvorrichtung zum Einführen eines medizinischen Implantats vorgeschlagen, welches durch eine Relativbewegung zwischen einem ersten und einem zweiten Einführelement freisetzbar ist, umfassend eine Freigabevorrichtung zum Lösen des medizinischen Implantats. Zwischen dem proximalen und dem distalen Ende ist ein Verkürzungsbereich vorgesehen, in dem bei Erzeugung einer gezielten Relativbewegung zwischen dem ersten und dem zweiten Einführelement der Einführvorrichtung eine Länge wenigstens eines der Einführelemente zwischen dem proximalen und dem distalen Ende verkürzbar ist, wobei der Verkürzungsbereich in einem Handgriff am proximalen Ende angeordnet ist.

Ein Zusammenfalten des Einführelements, z.B. des Außenschafts, ermöglicht die Ausgestaltung eines kurzen Handgriffs. Somit reduziert sich die Gesamtlänge des Katheters, was zu einem einfacheren Handling und einem verkürzten Verschiebungsweg des Katheters über den Führungsdraht führt. Zur Freigabe des Stents braucht der Anwender nur eine Hand einzusetzen.

Gemäß einer günstigen Ausgestaltung kann wenigstens eines der Einführelemente einen geschlitzten Bereich aufweisen. Der geschlitzte Bereich kann ein oder mehrere geschlitzte Segmente aufweisen, mit wenigstens einem axialen Schlitz entlang seiner axialen Erstreckung.

Gemäß einer günstigen Ausgestaltung kann wenigstens eines der Einführelemente einen geschlitzten Bereich aufweist mit wenigstens zwei axialen Schlitzen in Umfangsrichtung aufweist. Die Schlitze können sich z.B. diametral gegenüberliegen oder es können mehrere Schlitze, insbesondere symmetrisch, um die Mittelsehne des Einführelements angeordnet sein. Die Zahl der Schlitze um die Mittelsehne und/oder in axialer Richtung kann zweckmäßigerweise abhängig von einem oder mehreren Parametern, wie etwa Material und Dicke des Einführelements sowie Länge des Implantats, gewählt werden. Beispielsweise ist ein Einführelement, bei dem sind zwei oder mehr geschlitzte Segmente in axialer Richtung aufeinander folgen, stabiler als ein Einführelemente mit nur einem längeren geschlitzten Segment.

Gemäß einer günstigen Ausgestaltung kann der geschlitzte Bereich innerhalb des Handgriffs angeordnet sein. Das Einführelement, z.B. der Außenschaft, wird im ungeschlitzten Bereich zurückgezogen, und das Risiko eines gerissenen Außenschafts wird verringert.

Gemäß einer günstigen Ausgestaltung kann sich der geschlitzten Bereich in einen an den Handgriff angrenzenden Schaft erstrecken. Das Einführelement, z.B. der Außenschaft, wird im ungeschlitzten Bereich zurückgezogen, und das Risiko eines gerissenen Außenschafts wird verringert.

Die Erfindung ist nachfolgend beispielhaft, anhand von in Zeichnungen dargestellten Ausführungsbeispielen, näher erläutert. Es zeigen in schematischer Darstellung:
- Fig. 1: eine erste Ausgestaltung eines Einführvorrichtung nach einem Aspekt der Erfindung mit einer Freigabevorrichtung nach einem anderen Aspekt der Erfindung mit einem geschlitzten Bereich eines Außenschafts im proximalen Bereich innerhalb eines Handgriffs der Einführvorrichtung;
- Fig. 2: eine weitere Ausgestaltung eines Einführvorrichtung nach einem Aspekt der Erfindung mit einer Freigabevorrichtung nach einem anderen Aspekt der Erfindung mit einem geschlitzten Bereich eines Außenschafts im proximalen Bereich innerhalb eines Handgriffs der Einführvorrichtung und einem daran anschließendem Stabilisierungsschaft;
- Fig. 3: die Ausgestaltung nach Figur 2 mit teilweise zurückgezogenem Außenschaft;
- Fig. 4: eine Ausgestaltung ähnlich wie in Figur 1 mit teilweise zurückgezogenem Außenschaft und Elektromotor als Antrieb einer Aufwickeleinrichtung; und
- Fig. 5: eine weitere Ausgestaltung eines Einführvorrichtung nach einem Aspekt der Erfindung mit einer Freigabevorrichtung nach einem anderen Aspekt der Erfindung mit einem geschlitzten Bereich eines Außenschafts im proximalen Bereich innerhalb eines Handgriffs der Einführvorrichtung mit zwei Aufwickeleinheiten zum Aufwickeln des Außenschafts.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

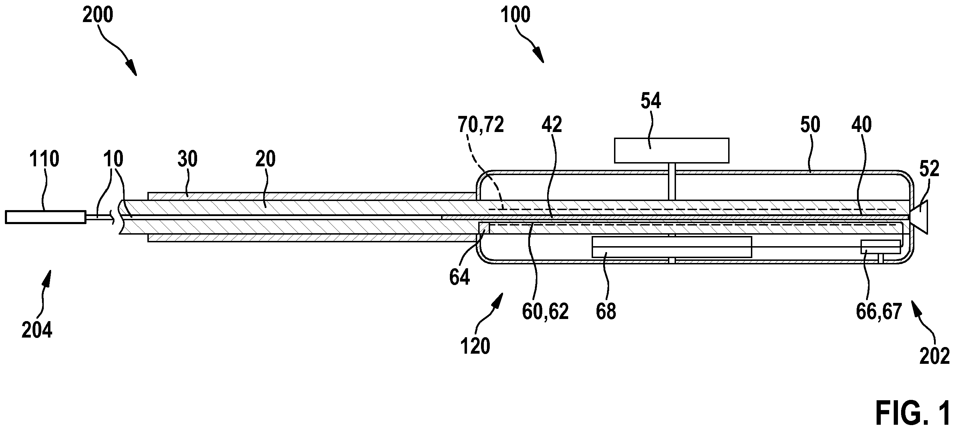

Figur 1 zeigt zur Erläuterung der Erfindung eine erste Ausgestaltung einer Einführvorrichtung 200 nach einem Aspekt der Erfindung in Form eines Katheters mit einer Freigabevorrichtung 100 nach einem anderen Aspekt der Erfindung. Die Freigabevorrichtung 100 dient zum Lösen eines medizinischen Implantats 110 von einer Einführvorrichtung 200, bei welcher das Implantat 110 durch eine Relativbewegung zwischen einem ersten Einführelement 10, z.B. einem Innenschaft 10, und einem zweiten Einführelement 20, z.B. einem Außenschaft 20, freisetzbar ist. Während des Einführens ist das Implantat 110 auf dem Innenschaft 10 angeordnet und vom Außenschaft 20 abgedeckt. Durch den Innenschaft 10 kann ein Führungsdraht (nicht dargestellt) geführt sein. Üblicherweise erfolgt das Freisetzen des Implantats 110 dadurch, dass der Außenschaft 20 gegenüber dem Innenschaft 10 zurückgezogen wird und das Implantat 110 freigelegt und am gewünschten Ort abgelegt wird.

Die Einführvorrichtung 200 weist ein proximales Ende 202 auf, das im Benutzungszustand einem Anwender zugewandt ist und ein distales Ende 204, das im Benutzungszustand vom Anwender entfernt ist. Das Implantat 110 ist schematisch am distalen Ende 204 der Einführvorrichtung 200 angedeutet. Zwischen dem proximalen Ende 202 und dem distalen Ende 204 ist ein Verkürzungsbereich 120 vorgesehen, in dem bei Erzeugung einer gezielten Relativbewegung zwischen dem Außenschaft 10 und dem Innenschaft 20 der Einführvorrichtung 200 eine Länge wenigstens eines der Einführelemente 10 oder 20, z.B. des Außenschafts 20, zwischen dem proximalen Ende 202 und dem distalen Ende 204 verkürzbar ist.

Am proximalen Ende 202 ist ein Handgriff 50 angeordnet, in dessen Inneren sich die Freigabevorrichtung 100 befindet. Eine Aufwickeleinrichtung 68 kann von außen mit einem Handrad 54 betätigt werden. In diesem Ausführungsbeispiel ist eine Zugeinrichtung 60 mit einem Zugfaden 62 vorgesehen, der am Außenschaft 20 befestigt und über eine Umlenkeinrichtung 66, z.B. eine Umlenkrolle 67, zu der Aufwickeleinrichtung 68 im Handgriff 50 geführt ist. Der Handgriff 50 kann mit einem üblichen Ventil 52, z.B. einem Luer-Lock, versehen sein, mit dem gleichzeitig das Führungsdrahtlumen im Innenschaft 10 und der Bereich zwischen dem Außenschaft 20 und dem Innenschaft 10 entlüftet werden kann.

Der Außenschaft 20 weist einen geschlitzten Bereich mit einem in axialer Richtung vorgeschlitzten Segment 70 auf, wobei der Zugfaden 62 am distalen Ende des vorgeschlitzten Segments 70 mit einer Befestigung 64 befestigt ist. Die Anzahl der vorgeschlitzten Segmente 70 ist je nach Dimension und Material des Außenschafts 20 zu wählen. Zweckmäßigerweise sind in dem oder den vorgeschlitzten Segmenten Schlitze 72 paarweise diametral gegenüberliegend ausgeführt. Es können auch mehr Schlitze 72 am Umfang angeordnet sein, z.B. drei oder mehr, die symmetrisch am Umfang verteilt sind. Im geschlitzten Bereich ist der Innenschaft 10 mit einem stabilisierenden Schaft 40, z.B. einem Metallschaft 42, umgeben.

Der Innenschaft 10 ist vom Außenschaft 20 umgeben. Nahe des Handgriffs 50 ist der Außenschaft 20 von einem Stabilisierungsschaft 30 umgeben. Der geschlitzte Bereich des Außenschafts 20 kann auch teilweise im Stabilisierungsschaft 30 des Katheters liegen. Der Stabilisierungsschaft 30 verhindert eine unerwünschte Faltung des Außenschafts 20 außerhalb des Handgriffs 50.

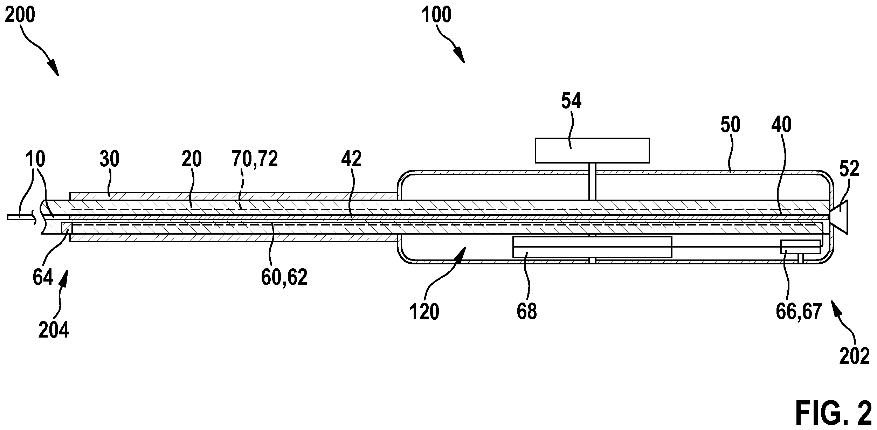

Figur 2 zeigt zur Erläuterung der Erfindung eine weitere Ausgestaltung einer Einführvorrichtung 200 nach einem Aspekt der Erfindung in Form eines Katheters mit einer Freigabevorrichtung 100 nach einem anderen Aspekt der Erfindung. Die Anordnung entspricht weitestgehend derjenigen in Figur 1, auf deren Beschreibung zur Vermeidung unnötiger Wiederholungen verwiesen wird.

Anders als in Figur 1 erstreckt sich der geschlitzte Bereich des Außenschafts 20 nunmehr auch im Stabilisierungsschaft 30, so dass beim Zurückziehen des Außenschafts 20 in den Handgriff 50 eine größere Länge zur Verfügung steht.

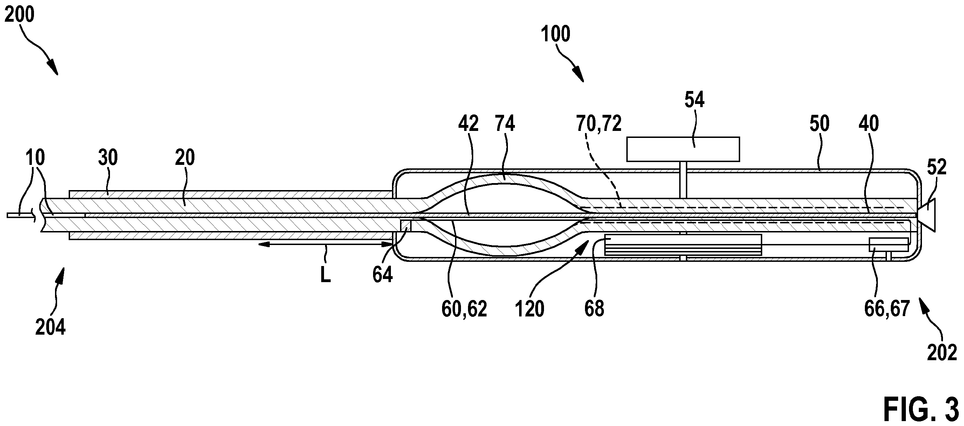

Figur 3 zeigt die Anordnung aus Figur 2 mit teilweise zurückgezogenem Außenschaft 20, wobei das Implantat (nicht dargestellt) bereits teilweise freigegeben ist. Der Außenschaft 20 ist um eine Länge L zurückgezogen. Hierdurch öffnet sich der Außenschaft 20 im geschlitzten Bereich, bildet zu beiden Seiten Ausbauchungen 74 und faltet sich bei weiterem Zurückziehen zusammen.

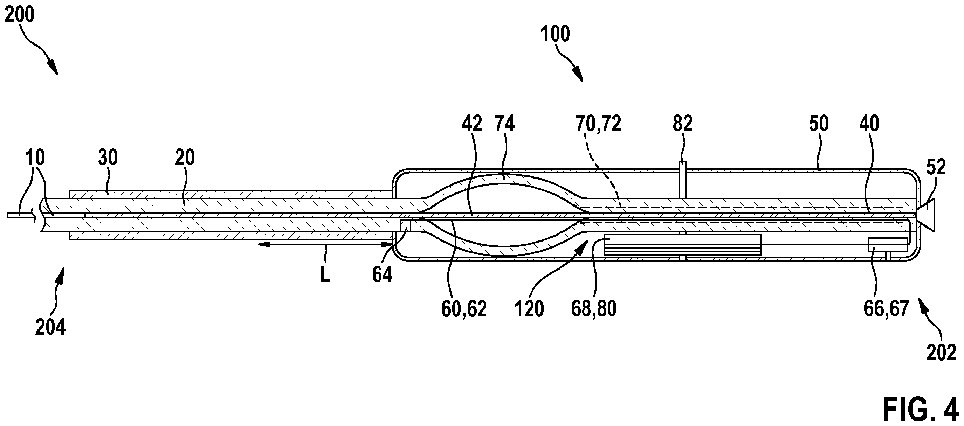

Figur 4 zeigt eine Variante entsprechend den Ausgestaltungen in Figur 1 und Figur 2, bei der das Handrad 54 durch einen Einschaltknopf 82 ersetzt ist und als Aufwickeleinrichtung 68 ein Motor eingesetzt ist. Dies erleichtert die Bedienung der Freigabevorrichtung 100 weiter. Es lassen sich hierdurch auch unterschiedlichen Aufwickelgeschwindigkeiten realisieren. Alternativ kann auch eine Aufwickeleinrichtung vorgesehen sein, die mit Hilfe von Federkraft gedreht wird.

Generell kann zum Aufwickeln des Zugfadens 62 auch eine Flaschenzug-Anordnung im Handgriff 50 mit zwei oder mehr Umlenkrollen 67 integriert sein. Dabei ist in bekannter Weise die Anzahl tragender Seile, auf die sich die Last verteilen kann, entscheidend für die Zugkraft, die der Anwender aufbringen muss. Verschiedenste Anordnungen von an sich bekannten Flaschenzügen können hierfür eingesetzt werden.

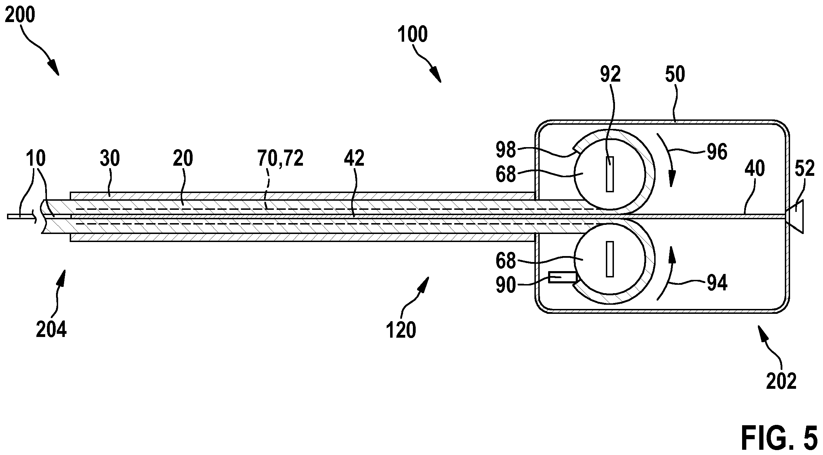

Figur 5 zeigt eine alternative Ausgestaltung der Erfindung, bei der die Aufwickeleinrichtung 68 wenigstens zwei symmetrisch am Katheter angeordnete Rollen umfassen, die synchron in gegenläufigen Drehrichtungen 94, 96 gedreht werden können, z.B. mit einem Griff 92 zum Drehen der Rollen. Hier können wenigstens zwei Hälften eines vorgeschlitzten Segments 70 des Außenschafts 20 in getrennte Flächenbereiche geteilt werden, die jeweils separat von der Aufwickeleinrichtung 68 gefasst und aufgewickelt werden können. Dies erleichtert das Aufwickeln, das mit weniger Kraftaufwand erfolgen kann.

Die Aufwicklung kann beispielsweise mit Hilfe eines Motors (z.B. Elektromotor) durchgeführt werden.

Die Hälften des vorgeschlitzten Segments 70 können an ihren Enden 98 mit den Rollen verbunden sein. Zur Arretierung der Rollen kann ein Sicherheitspin 90 vorgesehen sein, der gelöst werden kann, wenn der Außenschaft 20 zurückgezogen werden soll.

## Patentansprüche

1. Freigabevorrichtung (100) zum Lösen eines medizinisches Implantats (110) von einer Einführvorrichtung (200), bei welcher das Implantat (110) durch eine Relativbewegung zwischen einem ersten und einem zweiten Einführelement (10, 20) freisetzbar ist, wobei die Einführvorrichtung (200) ein proximales Ende (202) aufweist, das im Benutzungszustand einem Anwender zugewandt ist und ein distales Ende (204), das im Benutzungszustand vom Anwender entfernt ist, wobei zwischen dem proximalen und dem distalen Ende (202, 204) ein Verkürzungsbereich (120) vorgesehen ist, in dem bei Erzeugung einer gezielten Relativbewegung zwischen dem ersten und dem zweiten Einführelement (10, 20) der Einführvorrichtung (200) eine Länge wenigstens eines der Einführelemente (20) zwischen dem proximalen und dem distalen Ende (202, 204) verkürzbar ist, **dadurch gekennzeichnet, dass** das Verkürzen des Einführelements (20) mittels Zusammenfalten erfolgt.

2. Freigabevorrichtung nach Anspruch 1, **dadurch gekennzeichnet, dass** im Verkürzungsbereich (120) eine Aufwickeleinrichtung (68) vorgesehen ist.

3. Freigabevorrichtung nach Anspruch 1 oder 2, **dadurch gekennzeichnet, dass** eine Zugeinrichtung (60) vorgesehen ist, die an wenigstens einem der Einführelemente (20) angreift, um die Relativbewegung zwischen dem ersten und dem zweiten Einführelement (10, 20) zu bewirken.

4. Freigabevorrichtung nach Anspruch 3, **dadurch gekennzeichnet, dass** die Zugeinrichtung (60) einen Zugfaden (62) aufweist, der mit der Aufwickeleinrichtung (68) aufwickelbar ist.

5. Freigabevorrichtung nach einem der Ansprüche 3 oder 4, **dadurch gekennzeichnet, dass** die Aufwickeleinrichtung (68) eine Umlenkeinrichtung (66) aufweist, die mit der Zugeinrichtung (62) zusammenwirkt.

6. Freigabevorrichtung nach Anspruch 5, **dadurch gekennzeichnet, dass** die Umlenkeinrichtung (66) wenigstens zwei Umlenkrollen (67) aufweist.

7. Freigabevorrichtung nach Anspruch 1 oder 2, **dadurch gekennzeichnet, dass** wenigstens eines der Einführelemente (20) zur Verkürzung seiner Länge aufwickelbar ist.

8. Freigabevorrichtung nach einem der vorhergehenden Ansprüche, **dadurch gekennzeichnet, dass** die Aufwickeleinrichtung (68) mit einem Handrad (54) gekoppelt ist.

9. Freigabevorrichtung nach einem der vorhergehenden Ansprüche, **dadurch gekennzeichnet, dass** die Aufwickeleinrichtung (68) mit einem Motor (80) gekoppelt ist.

10. Freigabevorrichtung nach einem der vorhergehenden Ansprüche, **dadurch gekennzeichnet, dass** das Einführelement (20) im proximalen Bereich zusammengefaltet wird.

11. Einführvorrichtung (200) zum Einführen eines medizinischen Implantats (110), welches durch eine Relativbewegung zwischen einem ersten und einem zweiten Einführelement (10, 20) freisetzbar ist, umfassend eine Freigabevorrichtung (100) zum Lösen des medizinischen Implantats (110), nach einem der vorhergehenden Ansprüche, **dadurch gekennzeichnet, dass** zwischen dem proximalen und dem distalen Ende (202, 204) ein Verkürzungsbereich (120) vorgesehen ist, in dem bei Erzeugung einer gezielten Relativbewegung zwischen dem ersten und dem zweiten Einführelement (10, 20) der Einführvorrichtung (200) eine Länge wenigstens eines der Einführelemente (20) zwischen dem proximalen und dem distalen Ende (202, 204) mittels Zusammenfalten des Einführelements verkürzbar ist, wobei der Verkürzungsbereich (120) in einem Handgriff (50) am proximalen Ende (202) angeordnet ist.

12. Einführvorrichtung nach Anspruch 11, **dadurch gekennzeichnet, dass** wenigstens eines der Einführelemente (20) einen geschlitzten Bereich aufweist mit wenigstens einem geschlitzten Segment (70) entlang seiner axialen Erstreckung.

13. Einführvorrichtung nach Anspruch 11 oder 12, **dadurch gekennzeichnet, dass** wenigstens eines der Einführelemente (20) einen geschlitzten Bereich aufweist mit wenigstens zwei axialen Schlitzen (72) in Umfangsrichtung aufweist.

14. Einführvorrichtung nach einem der Ansprüche 11 bis 13, **dadurch gekennzeichnet, dass** der geschlitzte Bereich innerhalb des Handgriffs (50) angeordnet ist.

15. Einführvorrichtung nach einem der Ansprüche 12 bis 14, **dadurch gekennzeichnet, dass** sich der geschlitzten Bereich in einen an den Handgriff (50) angrenzenden Schaft (30) erstreckt.

## Claims

1. A release device (100) for releasing a medical implant (110) from an insertion device (200), in which the implant (110) can be released by a relative movement between a first and a second insertion element (10, 20), wherein the insertion device (200) has a proximal end (202), which faces the user during use, and a distal end (204), which is distanced from the user during use, wherein, between the proximal and the distal end (202, 204), a shortening region (120) is provided, in which, when generating a targeted relative movement between the first and the second insertion element (10, 20) of the insertion device (200), a length of at least one of the insertion elements (20) between the proximal end and the distal end (202, 204) can be shortened, **characterised in that** the insertion element (20) is shortened by folding.

2. The release device according to claim 1, **characterised in that** a winding arrangement (68) is provided in the shortening region (120).

3. The release device according to claim 1 or 2, **characterised in that** a traction arrangement (60) is provided which acts on at least one of the insertion elements (20) in order to implement a relative movement between the first and the second insertion element (10, 20).

4. The release device according to claim 3, **characterised in that** the traction arrangement (60) comprises a traction thread (62), which can be wound up using the winding arrangement (68).

5. The release device according to one of claims 3 or 4, **characterised in that** the winding arrangement (68) comprises a deflection arrangement (66), which cooperates with the traction arrangement (62).

6. The release device according to claim 5, **characterised in that** the deflection arrangement (66) comprises at least two deflection rolls (67).

7. The release device according to claim 1 or 2, **characterised in that** at least one of the insertion elements (20) can be wound up to shorten the length thereof.

8. The release device according to one of the preceding claims, **characterised in that** the winding arrangement (68) is coupled to a handwheel (54).

9. The release device according to one of the preceding claims, **characterised in that** the winding arrangement (68) is coupled to a motor (80).

10. The release device according to any one of the preceding claims, **characterised in that** the insertion element (20) is folded in the proximal region.

11. An insertion device (200) for inserting a medical implant (110), which can be released by a relative movement between a first and a second insertion element (10, 20), comprising a release device (100) for releasing the medical implant (110) according to one of the preceding claims, **characterised in that**, between the proximal and the distal end (202, 204), a shortening region (120) is provided, in which, when generating a targeted relative movement between the first and the second insertion element (10, 20) of the insertion device (200), a length of at least one of the insertion elements (20) between the proximal and the distal end (202, 204) can be shortened by folding the insertion element, wherein the shortening region (120) is arranged in a handle (50) at the proximal end (202).

12. The insertion device according to claim 11, **characterised in that** at least one of the insertion elements (20) comprises a slitted region with at least one slitted segment (70) along the axial extent thereof.

13. The insertion device according to claim 11 or 12, **characterised in that** at least one of the insertion elements (20) has a slitted region with at least two axial slits (72) in the peripheral direction.

14. The insertion device according to one of claims 11 to 13, **characterised in that** the slitted region is arranged within the handle (50).

15. The insertion device according to one of claims 12 to 14, **characterised in that** the slitted region extends into a shaft (30) bordering the handle (50).

## Revendications

1. Dispositif de dégagement (100) pour la libération d'un implant (110) médical d'un dispositif d'insertion (200), chez lequel l'implant (110) peut être dégagé par un mouvement relatif entre un premier et un deuxième élément d'insertion (10, 20), où le dispositif d'insertion (200) présente une extrémité proximale (202), qui est orientée vers un utilisateur dans l'état d'utilisation, et une extrémité distale (204), qui est éloignée de l'utilisateur dans l'état d'utilisation, où une zone de raccourcissement (120) est prévue entre l'extrémité proximale et l'extrémité distale (202, 204) en ce que, lors de la réalisation d'un mouvement relatif ciblé entre le premier et le deuxième élément d'insertion (10, 20) du dispositif d'insertion (200), une longueur d'au moins un des éléments d'insertion (20) peut être diminuée entre l'extrémité proximale et l'extrémité distale (202, 204), **caractérisé en ce que** le raccourcissement de l'élément d'insertion (20) est effectuée au moyen d'un repliement.

2. Dispositif de dégagement selon la revendication 1, **caractérisé en ce qu'**un dispositif d'enroulement (68) est prévu dans la zone de raccourcissement (120).

3. Dispositif de dégagement selon la revendication 1 ou 2, **caractérisé en ce qu'**un dispositif de traction (60) est prévu qui est en prise avec au moins l'un des éléments d'insertion (20) afin de provoquer le mouvement relatif entre le premier et le deuxième élément d'insertion (10, 20).

4. Dispositif de dégagement selon la revendication 3, **caractérisé en ce que** le dispositif de traction (60) présente un fil de traction (62) qui peut être enroulé avec le dispositif d'enroulement (68).

5. Dispositif de dégagement selon l'une des revendications 3 ou 4, **caractérisé en ce que** le dispositif d'enroulement (68) présente un dispositif de dérivation (66) qui agit conjointement avec le dispositif de traction (62).

6. Dispositif de dégagement selon la revendication 5, **caractérisé en ce que** le dispositif de dérivation (66) présente au moins deux rouleaux de dérivation (67).

7. Dispositif de dégagement selon la revendication 1 ou 2, **caractérisé en ce qu'**au moins l'un des éléments d'insertion (20) peut être enroulé pour la diminution de sa longueur.

8. Dispositif de dégagement selon l'une des revendications précédentes, **caractérisé en ce que** le dispositif d'enroulement (68) est couplé avec un volant de manoeuvre manuel (54).

9. Dispositif de dégagement selon l'une des revendications précédentes, **caractérisé en ce que** le dispositif d'enroulement (68) est couplé avec un moteur (80).

10. Dispositif de dégagement selon l'une des revendications précédentes, **caractérisé en ce que** l'élément d'insertion (20) est replié dans la zone proximale.

11. Dispositif d'insertion (200) permettant l'insertion d'un implant (110) médical, lequel peut être dégagé par un mouvement relatif entre un premier et un deuxième élément d'insertion (10, 20), comprenant un dispositif de dégagement (100) pour la libération de l'implant (110) médical selon l'une des revendications précédentes, **caractérisé en ce qu'**une zone de raccourcissement (120) est prévue entre l'extrémité proximale et l'extrémité distale (202, 204) **en ce que**, lors de la réalisation d'un mouvement relatif ciblé entre le premier et le deuxième élément d'insertion (10, 20) du dispositif d'insertion (200), une longueur d'au moins un des éléments d'insertion (20) peut être diminuée entre l'extrémité proximale et l'extrémité distale (202, 204) au moyen d'un repliement de l'élément d'insertion, où la zone de raccourcissement (120) est aménagée dans une poignée manuelle (50) à l'extrémité proximale (202).

12. Dispositif d'insertion selon la revendication 11, **caractérisé en ce qu'**au moins un des éléments d'insertion (20) présente une zone fendue avec au moins un segment (70) fendu le long de son extension axiale.

13. Dispositif d'insertion selon la revendication 11 ou 12, **caractérisé en ce qu'**au moins un des éléments d'insertion (20) présente une zone fendue avec au moins deux fentes (72) axiales dans la direction circonférentielle.

14. Dispositif d'insertion selon l'une des revendications 11 à 13, **caractérisé en ce que** la zone fendue est aménagée à l'intérieur de la poignée manuelle (50).

15. Dispositif d'insertion selon l'une des revendications 12 à 14, **caractérisé en ce que** la zone fendue s'étend dans une tige (30) limitrophe de la poignée manuelle (50).
